# EUROPEAN PATENT APPLICATION

(11) **EP 3 995 128 A1**
(43) Date of publication of application: **11.05.2022**
(21) Application number: 20834879.7
(22) Date of filing: 03.07.2020
(51) Int. Cl.: A61K 8/9783, A61Q 7/02, A61Q 17/00, A61Q 19/08, A61K 36/18

(54) **METHOD FOR OBTAINING BIOACTIVE INGREDIENTS, USE OF SUBCRITICAL-WATER EXTRACTION PROCESS, BIOACTIVE INGREDIENT, USE OF BIOACTIVE INGREDIENT AND COSMETIC COMPOSITION**

(30) Priority: 04.07.2019 BR 102019013936
(71) Applicant: Natura Cosméticos S.A., 05106-000 São Paulo SP (BR)
(72) Inventor: SANTOS DE SOUZA, Ana Karoliny, 07790-190 Cajamar - SP (BR); BRÁS COSTA, Camila, 07790-190 Cajamar - SP (BR); ZIEGLER STÜKER, Caroline, 07790-190 Cajamar - SP (BR); FERRARI, Cintia Rosa, 07790-190 Cajamar - SP (BR); DE OLIVEIRA, Daniel Henrique, 07790-190 Cajamar - SP (BR); ZIMBARDI, Daniela, 07790-190 Cajamar - SP (BR); SHIGERU TAKANO, Felipe, 07790-190 Cajamar - SP (BR); ANDRADE ARCURI, Helen, 07790-190 Cajamar - SP (BR); BELTRAME REIGADA, Juliana, 07790-190 Cajamar - SP (BR); CARVALHÃES LAGO NOLD, Juliana, 07790-190 Cajamar - SP (BR); ARROTEIA, Kelen Fabiola, 07790-190 Cajamar - SP (BR); DIBBERNN GANZERLA, Melissa, 07790-190 Cajamar - SP (BR); FERRÃO QUEIROZ, Nian Iury, 07790-190 Cajamar - SP (BR); CUNHA DE ASSIS CASTRO, Rafael, 07790-190 Cajamar - SP (BR); RABELO SCHEFER, Renata, 07790-190 Cajamar - SP (BR); BEZERRA CLAUDIO DE AVILA, Thais, 07790-190 Cajamar - SP (BR); ABRAHÃO DIAS, Ana Luisa, 05106-000 São Paulo - SP (BR)
(74) Representative: Simmons & Simmons
(86) International application number: PCT/BR2020/050244
(87) International publication number: WO 2021/000037

(57) **Abstract**

The present invention deals with a process for obtaining bioactive ingredients, the use of a specific extraction process and new bioactive ingredients from renewable sources, particularly from Amazonia, which can be used in the preparation of cosmetic compositions for treating the skin and hair and/or scalp. Said bioactive ingredients are achieved by a subcritical water extraction process parameterized to achieve bioactive compounds from different species and different plant parts.

## Description

### FIELD OF THE INVENTION

The present invention deals with a process for obtaining bioactive ingredients, the use of a specific extraction process and new bioactive ingredients from renewable sources, particularly from Amazonia, which can be used in the preparation of cosmetic compositions for treating the skin and hair and/or scalp. Said bioactive ingredients are achieved by a subcritical water extraction process parameterized to achieve bioactive compounds from different species and different plant parts.

### BACKGROUND OF THE INVENTION

Plants have numerous chemical compounds in their parts such as roots, stems, leaves, flowers, fruits and seeds with highly attractive properties capable of providing unexpected cosmetic benefits.

Thus, the elements of biodiversity are a rich source for obtaining new cosmetic raw materials. However, its potential has still been untapped.

This is because despite the state of the art suggests a variety of extraction processes, such as maceration, percolation, Soxhlet extraction, infusion, decoction, ultrasound extraction and extraction by sub and supercritical fluids, there still exists a need for improved and more effective processes, which effectively provide ingredients with a high content of bioactive compounds exhibiting proven biological effectiveness, particularly in the skin, hair or scalp.

In general, the process of obtaining new cosmetic raw materials from the biodiversity faces several challenges. In many industrial processes, the initial phase of preparing a product requires the use of specific techniques suited to different plant matrices. In addition, the various extraction methods have to take effectiveness and selectivity into account, which factors are important for obtaining cosmetic ingredients that are proven to be bioactive, since the chemical composition of plant matrices is quite complex and various types of compounds, non-bioactive biological agents or, eventually, even harmful compounds can be extracted.

In this scenario, at the expense of economic implications, the development of new extraction techniques has been greatly promoted in recent years due to environmental requirements, public health regulations and the need to minimize increasingly strict energy costs.

There is an interest for extraction processes using sub- and supercritical fluids, mainly in the recovery of highly pure bioactive compounds. However, although in recent years it has been characterized as an emerging technology due to its several advantages over conventional extraction methods, such as the fact that it is a clean and selective technology, the high investment cost in installing the sub and supercritical extraction plant is still a major obstacle to the industrial use of the technology.

Thus, there is still a challenge for achieving new efficient bioactive ingredients for use in the cosmetic industry, particularly for the treatment of the skin, hair and/or scalp.

### DESCRIPTION OF THE INVENTION

Seeking to overcome the obstacles of the prior art, particularly in relation to the complex chemical nature of different plant species, environmental requirements and, in particular, the processing efficiency, the present invention provides a new process for obtaining bioactive ingredients, particularly from renewable sources from Amazonia, which can be used in the preparation of cosmetic compositions for the treatment of the skin, hair and/or scalp.

Thus, the object of the present invention is a process for obtaining bioactive ingredients, which can be referred to as a green process, as it involves waste management and uses no organic solvents.

The process for obtaining the bioactive ingredients of the present invention comprises the following steps:
a) drying at least part of a plant species;
b) extracting bioactive ingredients from the plant species of interest by means of a subcritical water extraction process in a pressurized reactor, at a pressure of about 1 to 210 bars, for a time of 15 to 120 minutes, at a temperature of 100 to 200°C, comprising a solid:liquid ratio of from about 1:0.5 to about 1:14; and
c) optionally drying the bioactive ingredients obtained.

In a particular embodiment, the step a) of initial drying is carried out in wooden drying houses with air circulation exhaust systems. These are handmade small houses for drying plant parts such as leaves and/or waste, which are cheap to produce and, therefore, industrially viable.

In another particular embodiment, the step b) of extraction takes place under a specific parameterization of about 1.5 to about 4 bars of pressure, for a period of time of about 30 minutes, a temperature of from 140-160°C, and a solid:liquid ratio of about 1:7.

Moreover, step c) of drying can take place with or without the use of a drying agent, for example Aerosil, maltodextrin and starch.

It is a continuous process, which does not require the use of organic solvents, has shorter operating time, low production costs, high yield (throughput), in addition to providing superior active ingredients, that is, obtaining an extract without any chlorophyll and a higher content of bioactive compounds.

In a further aspect, the present invention refers to the use of a specific subcritical water extraction process to achieve high quality bioactive ingredients from plants, wherein extraction takes place in a pressurized reactor at a pressure of 1 to 210 bar, for a time of 15 to 120 minutes, at a temperature of 100 to 200°C, comprising a solid:liquid ratio of from about 1:0.5 to about 1:14.

Quality of the active ingredient obtained according to the present invention, in particular its high content of bioactive compounds, regardless of the origin and nature of the plant species, provides an effective cosmetic performance, that is, it allows the ingredient to act in different mechanisms of action in the skin, hair and scalp.

Unlike conventional subcritical water extraction processes, which usually use high pressure capsules and extracts are obtained at 300 bars, the process according to the present invention uses much lower pressures, allowing the use of different equipment such as reactors. A reduced pressure also means reduced energy expenditure.

Furthermore, in the process according to the present invention, reducing the amount of water used is very important as it reduces the energy expenditure in drying the extract and uses less water.

By "bioactive ingredients" in the context of the present invention is meant the product obtained by the process described herein, such as extracts.

The obtained bioactive ingredients comprise the set of bioactive substances or compounds that have a beneficial effect on the skin, hair or scalp.

In the context of the present invention, "bioactive substance or compound" is meant molecules that either alone or in combination with others are able to provide cosmetic benefits on the skin, hair or scalp.

In particular, the plant species useful for obtaining the bioactive ingredients according to the present invention are: *Byrsonima crassifolia* (nanche), *Inga edulis* (ice-cream-bean), *Libidibia férrea* (Brazilian ironwood), *Talinum triangulare* (waterleaf), *Solanum sessiliflorum* (cocona), *Costus arabicus* (canarana), *Euterpe oleraceae* (açaí palm), *Spondias mombin* (yellow mombin), *Elaeis guineenses* (oil palm) *e*/*ou Ananas erectifolius* (curaua).

In a particular embodiment, the bioactive ingredients are obtained from specific plant parts. Without imposing any limitation, for example, they can be obtained from aerial plant parts, such as stalks, leaves, flowers and fruits, or just leaves, as well as from agro-industrial residues such as seeds, mesocarp fiber or mucilage.

The bioactive ingredients according to the present invention are suitable for cosmetic use, for example, in the preparation of cosmetic compositions useful in the treatment of skin, hair and/or scalp.

The cosmetic compositions according to the present invention can provide benefits to the skin, hair and/or scalp such as anti-aging, antioxidant, anti-inflammatory, hydration, integrity/barrier function, pigmentation, cell renewal, protection against external agents, renewal/regeneration, actuation on the neuroendocrine/circadian rhythm system, anti-hair loss, hair growth control. These benefits are proven by gene expression screening in cell culture, bioinformatics analysis and identification of biological mechanisms.

The bioactive ingredients according to the present invention are obtained by subcritical water extraction parameterized to obtain bioactive compounds from different plant species.

There are several biochemical and biological tests to assess the biological potential of bioactive compounds, depending on the specific activity of interest to be evaluated. For example, the antioxidant activity can be assessed by several biochemical methods that contemplate different chemical mechanisms of antioxidant activity. This is because antioxidants can act by various mechanisms, for example, scavenging free radicals, breaking down peroxides and chelating metal ions; in addition to biological assays comprising stimulation of the endogenous production of antioxidant enzymes.

The technology chosen to assess the biological potential of extracts from selected plants according to the present invention involved a gene expression study of 368 genes followed by the identification of the biological mechanisms modulated by each extract.

To demonstrate the performance of each extract in the previously identified biological mechanisms, protein quantification of some specific markers or specific functional assays was performed, according to the potential identified for each sample.

After performing gene expression assays, the biological mechanisms modulated to provide skin benefits were identified and are presented below:
- Anti aging: Firmness (collagen, TIMPI, MMPI), protection of the elastic system (LOX, TIMP2), extracellular matrix (versican, heparan sulfate, biglican, lumican, matrilin, sindecan), dermo-epidermal junction, elastin production, increased epidermis thickness (via IGFI), elastic system, cell renewal, stimulation via MTOR and sirtuins;
- Antioxidant: Mitochondrial biogenesis (energy), oxidative stress response, autophagy/detoxification;
- Anti-inflammatory: Dermo calming effect;
- Hydration: Aquaporin synthesis, balanced desquamation, NMF synthesis;
- Integrity and Barrier Function: Lipid synthesis (fatty acids, ceramides), Adhesion/communication, Transport of molecules/nutrients, adhesion by keratins;
- Pigmentation: Melanogenesis inhibition (MCIR, MITF, HTR2A)
- Protection against external agents: Production of Antimicrobial Peptides, and Microbial Signaling;
- Renewal/Regeneration: Scarring, Cellular Renewal;
- Neuroendocrine System/Circadian Rhythm: Reduction of melatonin degradation/skin circadian control (serotoninergic and melatoninergic systems), stimulation to melatonin degradation (serotoninergic and melatonergic system), production of serotonin and receptors (serotoninergic and melatonergic system), beneficial effects of sunlight and well-being (increased receptors in UV-sensitive keratinocytes), stimulation of norepinephrine production (adrenergic system), stimulation of the barrier reinforcement under stress conditions (proliferation, differentiation, keratinocyte immune response), barrier homeostasis via association with the microbiota (increased dopamine receptors - dopaminergic system), stimulation of the action of vitamin D (increased receptor- secosteroidal axis), stimulation of vitamin D production (secosteroid axis), reduction of skin stress (HHA axis) (corticoid neuropeptides), reduced stress response/balance (HHA system), reduction of skin sensitization and itching reduction (endocannabinoid system), reduced norepinephrine production (adrenergic system) melanocytes have increased norepinephrine receptors under UV exposure, inhibition of melanogenesis activity (under sun exposure), reduced production of sebum - via decreased testosterone action (AR, HSD17B2) and reduced differentiation of sebaceous glands (PPARG), reduction of sensitization/allergy (histamine degradation - histamine cascade), increased histamine receptors, production of corticoid neuropeptides - reinforcement against skin stress (HHA axis) - Hormesis.

After performing gene expression assays, the modulated biological mechanisms in the hair/scalp were identified and are presented below:
- Anti-hair loss for both woman and man;
- Restoration of hair strand pigmentation; and
- Hair growth control;
- Healthy hair growth;
- Physical properties of the strand (diameter, length, elasticity and strength);
- Capillary anti-aging;
- Greater distribution of nutrients to the hair bulb;
- Stronger and thicker hair; and
- Health maintenance against dandruff;

The following examples, without any limitation, describe the particular embodiments of the present invention.

### EXAMPLES

Example 1. Process for preparing and assessing the activity profile of the obtained bioactive ingredients.

*Byrsonima crassifolia, Inga edulis, Libidibia ferrea, Talinum triangulare, Solanum sessiliflorum, Costus arabicus, Euterpe oleraceae, Spondias mombin, Elaeis guineenses e*/*ou Ananas erectifolius* plant species were each subjected to the sub-critical extraction process as described above; eleven different bioactive ingredients were obtained, which had their effects assessed in a *gene screening* in cell culture.

For this purpose, each of the species was subjected to prior drying in wooden drying houses with air exhaustion for an appropriate period of time. Then, the dry material was subjected to extraction by a subcritical water process in a pressurized reactor at a pressure of 2 bars, for a period of 30 minutes, at high temperature and a solid:liquid ratio of 1:7, 1:14 and 1 :1. The extracted material was dried with and without drying aid (Aerosil).

The process according to the present invention made it possible to obtain more concentrated and pure bioactive ingredients, having proven cosmetic efficacy by acting on several biological mechanisms related to the skin, hair and scalp.

Example 2. The cosmetic composition according to the present invention as a body moisturizer.

| Ingredient | Amount (%) |
|---|---|
| | Minimum-maximum |
| Water | 60-95 |
| caprylic/capric triglyceride | 2-10 |
| Cetyl Alcohol | 2-10 |
| Glycerin | 2-10 |
| Glyceryl Stearate Citrate | 2-10 |
| Hydroxyacetophenone | 0.1-2 |
| *Theobroma grandiflorum* seed butter | 2-10 |
| Tocopherol | 0.01-1 |
| *Bertholletia excelsa* seed oil | 1-6 |
| Polyglyceryl-3 Caprylate | 0.01-1 |
| Sodium gluconate | 0.01-1 |
| Sodium Polyacrylate | 0.01-1 |
| Propanediol | 1-5 |
| *Talinum triangulare* aerial parts extract | 0.01-5 |
| *Euterpe oleraceae* seed extract | 0.01-5 |

Example 3. Cosmetic composition according to the present invention for body hydration for dry skin.

| Ingredient | Amount (%) |
|---|---|
| | Minimum-maximum |
| Water | 60-95 |
| Dimethiconol/Cyclopentasiloxane | 1-5 |
| Dimethicone | 0.1 to 3 |
| disodium EDTA | 0.01-2 |
| *Elaeis guineensis* oil (palm) | 1-5 |
| Glycerin | 2-10 |
| *Heliantus Annuus* Oil | 1-5 |
| Cyclopentaxiloxane and Dimethiconol | 1 to 3 |
| Dimethicone | 0.5-2 |
| Disodium EDTA | 0.1-0.5 |
| Sodium Acrylate Copolymer | 0.5-3.0 |
| Hydroxyacetophenone | 0.01-2 |
| Lauryl glucoside/ Polyglyceryl-2 dipolyhydroxystearate/ Glycerin/ Water/ Citric acid | 0.01-2 |
| *Linum usitatissimum* seed oil | 0.01-2 |
| *Theobroma cacao* seed butter | 0.01-2 |
| Olus Oil/Tocopherol | 1-5 |
| Pentaerythrityl tetra-di-t-butyl hydroxyhydrocinnamate | 0.01-2 |
| Phenoxyethanol | 0.01-2 |
| Polyglyceryl-3 Caprylate | 0.01-2 |
| Sodium acrylate copolymer | 0.01-2 |
| Sodium Polyacrylate | 0.01-2 |
| Tocopheryl acetate | 0.01-2 |
| Trehalose | 0.01-2 |
| Fragrance | 0.1 to 1.5 |
| Maize starch | 0.01-2 |
| *Talinum triangulare* aerial parts extract | 0.01-5 |
| *Euterpe oleraceae* seed extract | 0.01-5 |

Example 4. Hair care cosmetic composition

| Ingredient | Amount (%) |
|---|---|
| | Minimum-maximum |
| Water | 70-95 |
| Behentrimonium chloride | |
| Cetearyl Alcohol | 0-10 |
| Cetyl ester | 0-5 |
| Citric acid | 0-10 |
| Decyl cocoate | 0-10 |
| Distearoylethyl Dimonium Chloride/Cetearyl Alcohol | 0-10 |
| Perfume | 0-5 |
| Glycerin | 0-10 |
| Glyceryl caprylate | 0-5 |
| Hydroxypropyl guar | 0-5 |
| Isoamyl laurate/ Propylene glycol diheptanoate | 0-10 |
| Lactic acid | 0-5 |
| *Astrocarium murumuru* seed butter | 0-10 |
| Sodium gluconate | 0-5 |
| Sodium hydroxide | 0-5 |
| Stearamidopropyl dimethylamine | 0-5 |
| *Elaeis Guineans* mesocarp fiber extract | 0.01 to 3 |

Example 5. Skin care cosmetic composition

| Ingredient | Amount (%) Minimum maximum |
|---|---|
| Water | 30-75 |
| Caprylic/capric triglyceride | 0.1-10 |
| Sodium gluconate | 0.05-012 |
| Glycerin | 1-8 |
| Glyceryl Stearate Citrate | 1-2 |
| Tocopheryl acetate | 0.1-2 |
| Xanthan gum | 0.22-0.33 |
| Perfume | 0.1-4 |
| *Inga edulis* leaf extract | 0.01-2 |
| *Talinum triangulare* aerial parts extract | 0.01-2 |

Example 6. Sunscreen Cosmetic Composition

| Ingredient | Amount (%) Minimum maximum |
|---|---|
| Water | 30-75 |
| Caprylic/capric triglyceride | 1-10 |
| Sodium gluconate | 0.05-012 |
| Glycerin | 1-8 |
| Potassium cetyl phosphate | 0.3-2.5 |
| Tocopheryl acetate | 0.1-2 |
| Xanthan Gum | 0.22-0.33 |
| Bis-ethylhexyloxyphenol methoxyphenyl triazine | 0.1-5 |
| Homosalate | 0.1-10 |
| Methylene Bis-Benzotriazolyl Tetramethylbutylphenol | 0.1-5 |
| Titanium dioxide | 0.1-20 |
| Zinc oxide | 0.1-20 |
| Ethylhexyl salicylate | 0.1-5 |
| *Inga Edulis* leaf extract | 0.01-2 |
| *Talinum triangulare* aerial parts extract | 0.01-2 |

The person skilled in the art will be able to readily assess through the teachings of the instant text and examples the advantages of the invention and to propose variations and equivalent alternatives of implementation without departing from the scope of the invention as defined in the appended claims.

## Claims

1. A PROCESS FOR OBTAINING BIOACTIVE INGREDIENTS, **characterized in that** it comprises the following steps:
a) drying at least part of a plant species;
b) extracting bioactive ingredients from the plant species by means of a subcritical water extraction process in a pressurized reactor, at a pressure of about 1 to 210 bars, for a time of 15 to 120 minutes, at a temperature of 100 to 200°C, comprising a solid:liquid ratio of from about 1:0.5 to about 1:14; and
c) optionally drying the bioactive ingredients obtained.

2. The PROCESS according to claim 1, **characterized in that** in step a) the drying of at least part of a plant species is carried out in wooden drying houses by air exhaustion by air circulation systems.

3. The PROCESS according to claim 1, **characterized in that** step b) of extraction by subcritical water in a pressurized reactor is carried out under a pressure of about 1.5 to about 4 bars, for a period of time of about 30 minutes, a temperature of from 140-160°C, and a solid:liquid ratio of about 1:7.

4. The PROCESS according to claim 1, **characterized in that** step c) of drying the bioactive ingredients is carried out with a drying agent.

5. The PROCESS, according to claim 1, **characterized in that** the plant species is selected from *Byrsonima crassifolia, Inga edulis, Libidibia ferrea, Guazuma ulmifolia, Talinum triagulare, Solanum sessiliflorum, Costus arabicus, Euterpe oleraceae, Spondias mombin, Elaeis guineenses and*/*or Ananas erectifolius.*

6. The PROCESS, according to claim 1, **characterized in that** the plant part is selected from aerial plant parts, such as stalks, leaves, flowers and fruits, and/or agro-industrial residues such as seeds, mesocarp fiber or mucilage fiber.

7. USE OF THE SUBCRITICAL WATER EXTRACTION PROCESS, **characterized in that** it is for obtaining bioactive ingredients from plant species, wherein the extraction takes place in a pressurized reactor at a pressure of from 1 to 210 bars, for a period of time of from 15 to 120 minutes, at a temperature of from 100 to 200°C, comprising the solid: liquid ratio of about 1:0.5 to about 1:14.

8. The USE, according to claim 7, **characterized in that** the pressure is 1.5 to 4 bars, the time is about 30 minutes, the temperature is 140-160°C, and the solid:liquid ratio is about 1:7.

9. The USE, according to claim 7, **characterized in that** the plant species is selected from *Byrsonima crassifolia, Inga edulis, Libidibia ferrea, Guazuma ulmifolia, Talinum triagulare, Solanum sessiliflorum, Costus arabicus, Euterpe oleraceae, Spondias mombin, Elaeis guineenses and*/*or Ananas erectifolius*

10. A BIOACTIVE INGREDIENT, **characterized in that** it comprises bioactive compounds, wherein the bioactive ingredient is obtained by the process as defined in any one of claims 1-6.

11. The USE OF A BIOACTIVE INGREDIENT, as defined in claim 10, **characterized in that** it is for the preparation of cosmetic compositions useful in the treatment of skin, hair and/or scalp.

12. The USE, according to claim 11, **characterized in that** the cosmetic compositions provide anti-aging, antioxidant, anti-inflammatory, hydration, integrity/barrier function, pigmentation, cell renewal, protection against external agents, renewal/ regeneration, activity on the neuroendocrine system/circadian rhythm, anti-hair loss and hair growth control.

13. A COSMETIC COMPOSITION, **characterized in that** it comprises at least one bioactive ingredient as defined in claim 10, and cosmetically acceptable carriers.

14. The COSMETIC COMPOSITION, according to claim 13, **characterized in that** it provides cosmetic benefits to the skin, hair and/or scalp, wherein the benefits are anti-aging, antioxidant, anti-inflammatory, hydration, integrity/barrier function, pigmentation, cell renewal, protection against external agents, renewal/regeneration, activity on the neuroendocrine system/circadian rhythm, anti-hair loss and hair growth control.
